# EUROPEAN PATENT SPECIFICATION

(11) **EP 2 631 289 B1**
(45) Date of publication and mention of the grant of the patent: **13.06.2018**
(21) Application number: 13156163.1
(22) Date of filing: 21.02.2013
(51) Int. Cl.: C11D 3/37, C11D 17/00, C11D 3/20, A61K 8/36, A61K 8/39, A61K 8/46, A61K 8/92, A61Q 5/12, A61Q 13/00, A61Q 17/00, A61Q 19/00, A61Q 19/08, A61K 8/02, A61K 8/11

(54) **Liquid laundry detergent comprising capsules II**
Flüssiges Waschmittel mit Kapseln II
Détergent liquide pour lessive comprenant des capsules II

(30) Priority: 24.02.2012 EP 12156899
(43) Date of publication of application: 28.08.2013
(73) Proprietor: Henkel AG & Co. KGaA, 40589 Düsseldorf (DE)
(72) Inventor: Marzouk, Ashraf, Cairo (EG); Jeschke, Rainer, 40595 Düsseldorf (DE); Nemmertz, Simone, 40699 Erkrath (DE); Ott, Thorsten, 41189 Mönchengladbach (DE)

(56) References cited:
- WO-A1-93/08248
- WO-A2-2010/048154
- DE-A1- 19 750 450

## Description

The present invention relates to a structured liquid detergent composition comprising surfactant, a structurant and a plurality of encapsulated actives. The present invention also describes a method for manufacturing such a liquid detergent composition.

Liquid detergents comprising microcapsules are very appealing to consumers. The inclusion of microcapsules in liquid detergents is desirable not only for aesthetic reasons but also for functional reasons such as isolation of incompatible ingredients, controlled and/or delayed release, etc. Ideally, the microcapsules are stably suspended in the liquid detergent and only dissolve/disintegrate in use.

Since consumers generally desire a clean and fresh odor whenever they open the package and smell the product, as well as at later points in their laundering experience such as a clean and fresh odor in the laundry room, and on laundered clothing, perfume microcapsules have been used in consumer products to improve fragrance deposition, retention and longevity.

One problem encountered with the production of liquid detergents comprising encapsulated actives is that the distribution of the encapsulated actives within the liquid matrix needs to be controlled so that the encapsulated actives do not overly float, sink or otherwise gravitate during processing, when packaged for later processing with other ingredients, or when in a packaged consumer product. In order to properly disperse and suspend the encapsulated actives with the liquid matrix, structuring agents can be introduced into the composition. There are number of compounds which can provide structuring benefits.

Known external structuring agents include polymers or gums such as gellan gum, alginate, carrageenan, xanthan gum, and guar gum. Although gums have been used to provide structuring benefits, the gums have been found to be undesirable due to any pH and electrolyte level sensitivity which can decrease their structuring capacity or lead to other undesirable issues such as composition opacity or gelling or clumping of product.

As disclosed in WO2010/048154 A2 bacterial celluloses or non-polymeric crystalline hydroxyl-functional materials also provide structuring benefits.

A drawback of many external structurants is their need for special processing conditions. In addition, many external structurants have to be used in high amounts in order to provide the desired structuring effect.

In addition, some external structurants are detrimental to the cleaning performance of the liquid detergent composition. Especially, graying of laundered clothing may be a severe problem that arises from the use of some polymeric structurants.

It is an object of the present invention to provide a liquid detergent composition comprising encapsulated actives, wherein the encapsulated actives are stably suspended, wherein the liquid detergent composition can be manufactured in an easy and cheap way and wherein the liquid detergent composition shows a good cleaning performance.

This object is achieved by a structured liquid detergent composition comprising:
(a) surfactant at a level of from 5 up to 60 % by weight of said composition, said surfactant comprising nonionic surfactant, anionic surfactant, zwitterionic surfactant, cationic surfactant and mixtures thereof;
(b) a structurant at a level of from 0.1 to 5 % by weight of said composition, said structurant comprising a ring opening product of an epoxidized fatty ester with a dihydric or polyhydric alcohol; and
(c) a plurality of encapsulated actives at a level of from 0.05 up to 1 % by weight of said composition, wherein the encapsulated actives have an d₉₀ value of below 50 µm.

It has surprisingly been found that the use of a ring opening product of an epoxidized fatty ester with said alcohol leads to a structured liquid detergent composition in which encapsulated actives can be stably suspended with a minimum amount of structurant.

It is preferred that said epoxidized fatty ester is selected from the group consisting of epoxidized fatty acid esters, epoxidized fatty acid triglycerides and mixtures thereof.

It has been shown that ring opening products of epoxidized fatty acid esters and/or epoxidized fatty acid triglycerides can be used as structurants - even in high amounts - without being detrimental to the cleaning performance of the liquid detergent composition.

Furthermore, it is preferred that the epoxidized fatty acid triglyceride is selected from the group consisting of epoxidized beef tallow, epoxidized palm oil, epoxidized peanut oil, epoxidized rapeseed oil, epoxidized cottonseed oil, epoxidized soybean oil, epoxidized sunflower oil, epoxidized linseed oil and mixtures thereof. Ring opening products of these epoxidized vegetable oils are oleochemical compounds derived from renewable raw materials.

In another preferred embodiment of the invention said alcohol is selected from the group consisting of ethane-1,2-diol, propane-1,2-diol, propane-1,3-diol, butane-1,4-diol, hexane-1,6-diol, neopentyl glycol, trimethylol propane, glycerol, trimethylol ethane, pentaerythritol, sorbitol, alkyl glycols, oligomeric glycols, oligomeric glycerols and mixtures thereof.

The specified structurant is very effective and, accordingly, it is preferred that the structurant is present at a level of from 0.15 % up to 2 % by weight of said composition and, preferably, at a level of from 0.5 % up to 1 % by weight of the liquid detergent composition.

In a yet another preferred embodiment of the invention encapsulated actives have a d₉₀ value of below 40 µm and most preferred a d₉₀ value of below 20 µm. With help of the specified structurants it is possible to stably suspend different sizes of encapsulated actives.

It is preferred that encapsulated actives comprise friable microcapsules, moisture-activated microcapsules, heat-activated microcapsules, or combinations thereof.

Depending on the use and the purpose of the encapsulated actives different types of encapsulation techniques are used. The liquid structured detergent composition is able to stably suspend these different types of microcapsules.

It is preferred that the encapsulated actives comprise an active selected from the group consisting of perfumes, softening agents, anti-static agents, refreshing agents, anti-microbial agents, disinfecting agents, anti-wrinkle agents, malodor control agents, insect/pet repellents, skin/fabric conditioning agents, silicones, anti-microbials, brighteners, bleaches, antifoams, and combinations thereof. In a more preferred embodiment of the invention the encapsulated actives comprises a perfume as an active.

Due to the physical or chemical characteristics of actives, actives may be incompatible with other compositional components of the liquid detergent composition or may be lost during post application processes such as rinsing or drying. By encapsulating actives their stability, delivery and release can be improved and controlled. Since a clean and fresh odor of the detergent composition and/or on items treated with the detergent composition is very important for consumers, by encapsulating perfumes an improved perfume delivery at all stages can be achieved.

The invention also relates to a method for manufacturing a structured liquid detergent composition comprising:
(a) surfactant at a level of from 5 up to 60 % by weight of said composition, said surfactant comprising nonionic surfactant, anionic surfactant, zwitterionic surfactant, cationic surfactant and mixtures thereof;
(b) a structurant at a level of from 0.1 to 5 % by weight of said composition, said structurant comprising a ring opening product of an epoxidized fatty ester with a dihydric or polyhydric alcohol;
(c) a plurality of encapsulated actives at a level of from 0.05 up to 1 % by weight of said composition,
wherein the structurant is added at a temperature above 50 °C.

It has been shown that an optimum structuring performance by a ring opening product of an epoxidized fatty ester with an alcohol as structurant can be achieved if the structurant is added at elevated temperatures in the manufacturing process. No other special processing conditions such as activation of the structurant by applying mechanical forces or pre-swelling are necessary.

The invention is described in greater detail below on the basis of examples, among other things.

In general, the present invention relates to a structured liquid detergent composition comprising surfactant, a ring opening product of an epoxidized fatty ester with a dihydric or polyhydric alcohol as structurant and a plurality of encapsulated actives, wherein the encapsulated actives have a d₉₀ value of below 50 µm.

The liquid detergent composition comprises surfactant at a level of from 5 up to 60 % by weight of said composition, preferably, at a level of from 10 up to 50 % by weight of said composition and, and even more preferred, at a level of from 15 up to 35 % by weight of said composition.

Said surfactant comprises nonionic surfactant, anionic surfactant, zwitterionic surfactant, cationic surfactant or mixtures thereof.

The anionic surfactant preferably comprises linear alkylbenzene sulphonate and/or fatty alcohol ether sulfate.

Fatty alcohol ether sulfates are water-soluble salts of the formula RO(A)ₘ SO₃M, in which R is an unsubstituted C₁₀-C₂₄-alkyl or -hydroxyalkyl radical, preferably a C₁₂-C₂₀-alkyl or - hydroxyalkyl radical, more preferably C₁₂-C₁₈-alkyl or -hydroxyalkyl radical. A is an ethylene oxide or propylene oxide unit, m is an integer greater than 0, preferably between approximately 0.5 and approximately 6, more preferably between approximately 0.5 and approximately 4, and M is a cation, for example sodium, potassium, lithium, calcium, magnesium, ammonium or a substituted ammonium cation. Specific examples of substituted ammonium cations are methyl-, dimethyl-, trimethylammonium and quaternary ammonium cations such as tetramethylammonium and dimethylpiperidinium cations, and also those which are derived from alkylamines such as ethylamine, diethylamine, triethylamine or mixtures thereof. Preferred examples include C₁₂-C₁₈ fatty alcohol ether sulfates where A is an ethylene oxide unit und the content of ethylene oxide units is 1, 2, 2.5, 3 or 4 mol per mole of the fatty alcohol ether sulfate, and in which M is sodium or potassium. Fatty alcohols with 1 to 4 ethylene oxide units, in particular 1 to 2 ethylene oxide units. A particularly preferred fatty alcohol ether sulfate is sodium lauryl ether sulfate with 2 ethylene oxide units.

The amount of fatty alcohol ether sulfate is preferably from 1 to 15 % by weight of said composition and more preferably from 2 to 10 % by weight of said composition.

The linear alkylbenzene sulphonate is preferably a linear alkylbenzene sulphonate having an alkyl chain length of C₈-C₁₅. In particular, the linear alkylbenzene sulphonate is a C₉-C₁₃ alkyl benzene sulphonate, a C₁₀-C₁₃ alkyl benzene sulphonate or a C₁₀-C₁₅ alkyl benzene sulphonate.

The amount of linear alkylbenzene sulphonate is preferably from 3 to 25 % by weight of said composition, more preferably from 5 to 18 % by weight of said composition and especially from 7 to 15 % by weight of said composition.

Further anionic surfactants that may additionally be present in the structured liquid detergent composition are fatty acid soaps. Saturated and unsaturated fatty acid soaps, such as the salts of lauric acid, myristic acid, palmitic acid, stearic acid, (hydrogenated) erucic acid, and behenic acid, are suitable, as are soap mixtures derived in particular from natural fatty acids, e.g. coconut, palm-kernel, olive-oil, or tallow fatty acids.

The amount of fatty acid soap is preferably from 1 to 8 % by weight of said composition, more preferably from 2,5 to 7 % by weight of said composition and especially from 4 to 6 % by weight of said composition.

The anionic surfactants, including the fatty acid soaps, can be present in the form of their sodium, potassium, or ammonium salts and as soluble salts of organic bases such as mono-, di-, or triethanolamine. The anionic surfactants are preferably present in the form of their sodium or potassium salts, in particular in the form of the sodium salts.

It may be preferred that the liquid detergent composition only contains fatty alcohol ether sulfates, linear alkylbenzene sulphonates and fatty acid soaps as anionic detergents.

The liquid detergent composition may comprise a nonionic surfactant at a level of from 5 % up to 30 % by weight of the liquid detergent composition.

The nonionic surfactants that can be used are by preference alkoxylated, advantageously ethoxylated, in particular primary alcohols having by preference 8 to 18 carbon atoms and an average of 1 to 12 mol ethylene oxide (EO) per mol of alcohol, in which the alcohol residue can be linear or preferably methyl-branched in the 2-position, or can contain mixed linear and methyl-branched residues, such as those that are usually present in oxo alcohol residues. Particularly preferred, however, are alcohol ethoxylates having linear residues made up of alcohols of natural origin having 12 to 18 carbon atoms, e.g. from coconut, palm, tallow, or oleyl alcohol, and an average of 2 to 8 EO per mol of alcohol. The preferred ethoxylated alcohols include, for example, C₁₂₋₁₄ alcohols with 3 EO, 4 EO, 5 EO, or 7 EO, C₉₋₁₁ alcohols with 7 EO, C₁₃₋₁₅ alcohols with 3 EO, 5 EO, 7 EO, or 8 EO, C₁₂₋₁₈ alcohols with 3 EO, 5 EO, or 7 EO, and mixtures thereof, such as mixtures of C₁₂₋₁₄ alcohol with 3 EO and C₁₂₋₁₈ alcohol with 7 EO. The degrees of ethoxylation indicated represent statistical averages, which can correspond to an integral or a fractional number for a specific product. Preferred alcohol ethoxylates exhibit a restricted distribution of homologs (narrow range ethoxylates, NRE). In addition to these nonionic surfactants, fatty alcohols with more than 12 EO can also be used. Examples of these are tallow fatty alcohol with 14 EO, 25 EO, 30 EO, or 40 EO. Nonionic surfactants that contain EO and PO groups together in the molecule are also usable according to the present invention. Block copolymers having EO-PO block units or PO-EO block units, but also EO-PO-EO copolymers or PO-EO-PO copolymers, can be used in this context. Also usable, of course, are mixed alkoxylated nonionic surfactants in which EO and PO units are distributed statistically rather than in block fashion. Such products are obtainable by the simultaneous action of ethylene oxide and propylene oxide on fatty alcohols. These nonionic surfactants are obtainable, for example, under the commercial name Dehydol® (from Cognis).

Also suitable nonionic surfactants are mixtures of a (more) branched ethoxylated fatty alcohol and a non-branched ethoxylated fatty alcohol, such as mixtures of C₁₆₋₁₈ fatty alcohol having 7 EO and 2-propylheptanol having 7 EO.

Further classes of nonionic surfactants used in preferred fashion are alkoxylated fatty acid alkyl esters, surfactants of the amine oxide type, polyhydroxy fatty acid amides or alkylpolyglucosides.

Most preferred the liquid detergent composition comprises a C₁₂₋₁₈ fatty alcohol having 7 EO or a C₁₃₋₁₅ oxo alcohol having 7 EO as nonionic surfactant.

Most preferred the liquid detergent composition comprises a mixture of nonionic and anionic surfactants.

It is essential for the present invention that the liquid detergent composition comprises a ring opening product of an epoxidized fatty ester with a dihydric or polyhydric alcohol as structurant. The structurant is present at a level of from 0.1 up to 5 % by weight of the liquid detergent composition. In a preferred embodiment the structurant is present at a level of 0.15 up to 2 % by weight of the liquid detergent composition and, in an even more preferred embodiment the structurants is present at a level of from 0.5 up to 1 % by weight of said composition.

Ring opening products of epoxidized fatty esters with said alcohols are oleo chemical compounds. Oleo chemical compounds are understood to be compounds based on natural oils and fats and include, for example, the reaction products of epoxidized fatty esters with monohydric, dihydric or polyhydric alcohols or glycerol esters of long-chain fatty acids which are at least partly substituted by hydroxyl groups .

A subsidiary group of these compounds are the ring-opening products of epoxidized triglycerides, i.e. epoxidized fatty acid glycerol esters which have been ring-opened with the ester bonds intact. The ring-opening products may be produced from a number of epoxidized triglycerides of vegetable or animal origin. For example, epoxidized triglycerides containing 2 to 10% by weight of epoxide oxygen are suitable. Products such as these may be obtained from a number of fats and oils, for example beef tallow, palm oil, peanut oil, rapeseed oil, cottonseed oil, soybean oil, sunflower oil and linseed oil, by epoxidation of the double bonds. Particularly preferred epoxidized triglycerides are epoxidized soybean oil and epoxidized linseed oil. In a particularly preferred embodiment the epoxidized triglyceride is epoxidized soybean oil.

In another preferred embodiment the epoxidized fatty ester is an epoxidized fatty methyl ester. Typical examples are the epoxides of palmitoleic acid methyl ester, oleic acid methyl ester, elaidic acid methyl ester, petroselic acid methyl ester, linoleic acid methyl ester or erucic acid methyl ester.

Suitable alcohols for the ring-opening of the epoxidized fatty esters are ethane-1,2-diol, propane-1,2-diol, propane-1,3-diol, butane-1,4-diol, hexane-1,6-diol, neopentyl glycol, trimethylol propane, glycerol, trimethylol ethane, pentaerythritol, sorbitol and hydroxy compounds containing ether groups, such as alkyl glycols, or oligomeric glycols and oligomeric glycerols. Diols such as ethane-1,2-diol, propane-1,2-diol, propane-1,3-diol, butane-1,4-diol, hexane-1,6-diol are preferably used for the ring-opening. Among the diols ethane-1,2-diol is preferably used for ring-opening.

The ring-opening reaction of epoxidized fatty acid esters with said alcohol may optionally be followed by transesterification with the starting material or with other esters subsequently added.

Suitable ring-opening products of epoxidized fatty acid esters with polyols can be purchased from Cognis under the name "Sovermol®". Sovermol® 750 and Sovermol® 1102 are preferably used as structurants.

After dissolving in water a ring opening product of an epoxidized fatty ester with said alcohol forms a network structure that is especially suited to stabilize and suspend small capsules, especially capsules having a d₉₀ value of below 50 µm.

The liquid detergent composition comprises a plurality of said encapsulated actives at a level of from 0.05 up to 1 % by weight of the liquid detergent composition. It is preferred that the encapsulated actives comprise an active selected from the group consisting of perfumes, softening agents, anti-static agents, refreshing agents, anti-microbial agents, disinfecting agents, anti-wrinkle agents, malodor control agents, insect/pet repellents, skin/fabric conditioning agents, silicones, anti-microbials, brighteners, bleaches, antifoams, and combinations thereof. In one embodiment the encapsulated active comprises a perfume as an active.

The preferred encapsulated actives have a d₉₀ value of below 40 µm and most preferred a d₉₀ value of below 30 µm.

The d₉₀ defines that equivalent diameter where 90 mass-% of the encapsulated actives have a smaller diameter.

In a very preferred embodiment of the invention the encapsulated actives have a diameter between 1 and 15 µm.

It is preferred that the encapsulated actives comprise friable microcapsules, moisture-activated microcapsules, heat- activated microcapsules, or combinations thereof.

The encapsulated actives preferably comprise friable microcapsules. "Friability" refers to the propensity of the microcapsules to rupture or break open when subjected to direct external pressures or shear forces. For purposes of the present invention, the microcapsules utilized are "friable" if, while attached to fabrics treated therewith, they can be ruptured by the forces. encountered when the capsule-containing fabrics are manipulated by being worn or handled (thereby releasing the contents of the capsule).

Although a preferred embodiment of the present invention is directed to actives encapsulated within friable microcapsules, the present invention is not being limited to only those microcapsules.

Typically, microcapsules comprise a spherical hollow shell of water insoluble or at least partially water insoluble material, typically polymer material, within which the active is contained.

Useful shell materials include materials selected from the group consisting of polyethylenes, polyamides, polystyrenes, polyisoprenes, polycarbonates, polyesters, polyacrylates, polyureas, polyurethanes, polyolefins, polysaccharides, epoxy resins, vinyl polymers, and mixtures thereof. Suitable shell materials include materials selected from the group consisting of reaction products of one or more amines with one or more aldehydes, such as urea cross-linked with formaldehyde or gluteraldehyde, melamine cross-linked with formaldehyde; gelatin-polyphosphate coacervates optionally cross-linked with gluteraldehyde; gelatin-gum Arabic coacervates; cross-linked silicone fluids; polyamine reacted with polyisocyanates and mixtures thereof. In a preferred embodiment, the shell material comprises melamine cross-linked with formaldehyde.

Microcapsules may be prepared using a range of conventional methods known to those skilled in the art for making shell capsules, such as interfacial polymerization, and polycondensation. With the help of these methods a thin polymer shell is created around droplets or particles of an active emulsified or dispersed in a carrier liquid.

Usually, the encapsulated actives are introduced into the liquid detergent composition in the form of an aqueous slurry of the microcapsules.

In a preferred embodiment, the active comprises a perfume. Non-limiting examples of suitable perfumes include blooming perfumes, perfume oils, and perfume raw materials comprising alcohols, ketones, aldehydes, esters, ethers, nitriles alkenes, and mixtures thereof.

In another preferred embodiment, the liquid detergent composition comprises an encapsulated perfume and a free perfume.

A general range of encapsulated active concentration, for example perfume microcapsules content, of the microcapsules slurry is between 1 and 50%, by weight, relative to the weight of the slurry, the latter also containing typically 4 to 20% by weight of encapsulating shell material in a free form, and the balance being water.

Preferably, the liquid detergent composition is a liquid laundry detergent composition or a liquid cleaning detergent composition and most preferred the liquid detergent composition is an aqueous liquid laundry detergent composition.

In addition to the surfactant, the structurant, and the plurality of encapsulated actives, a liquid detergent composition may contain further ingredients that further improve the applications-engineering or aesthetic properties of the liquid detergent composition. In the context of the present invention, the liquid detergent composition by preference additionally contains one or more substances from the group of the detergency builders, bleaching agents, bleach activators, bleach catalysts, enzymes, non-aqueous solvents, pH adjusting agents, free perfumes, fluorescing agents, dyes, hydrotopes, silicone oils, anti-redeposition agents, anti-gray agents, shrinkage preventers, wrinkle protection agents, dye transfer inhibitors, antimicrobial active substances, germicides, fungicides, antioxidants, preservatives, corrosion inhibitors, antistatic agents, bittering agents, ironing adjuvants, proofing and impregnation agents, swelling and anti-slip agents, softening compounds, complexing agents and UV absorbers.

From the above mentioned further ingredients detergency builders, enzymes, non-aqueous solvents, pH adjusting agents, free perfumes, fluorescing agents, dyes, silicone oils, soil-release polymers, anti-gray agents, dye transfer inhibitors, and preservatives are most preferred included into a liquid laundry detergent composition.

The liquid detergent composition, in particular the liquid laundry detergent composition, according to the present invention can be used to wash and/or clean textile fabrics.

The liquid detergent composition is manufactured using usual and known methods and processes. For example, the constituents of the liquid detergent composition can be simply mixed in agitator vessels, the water, non-aqueous solvent, and surfactants usefully being prepared first. The fatty acid component, if present, is then saponified at 50 to 60°C. Preferably, the structurant is added at this stage of the manufacturing process. After cooling under stirring the further constituents are then added in portions. In a final stage, the encapsulated actives are added and evenly distributed within the liquid detergent composition.

Table 1 below shows the composition of five liquid detergent compositions E1 to E5 according to the present invention. Quantities are indicated in wt% of active matter.

**Table 1:**

| | E1 | E2 | E3 | E4 | E5 |
|---|---|---|---|---|---|
| C₁₁-₁₃-alkylbenzenesulfonic acid, Na salt | 7 | 7 | 7 | 7 | 7 |
| Sodium lauryl ether sulfate with 2 EO | 9 | 9 | 9 | 9 | 9 |
| C₁₂₋₁₈ fatty alcohol with 7 EO | 7 | 7 | 7 | 7 | 7 |
| Coconut fatty acid, Na salt | 5.6 | 5.6 | 5.6 | 5.6 | 5.6 |
| Structurant 1 | 1 | 1 | 1 | 1 | -- |
| Structurant 2 | -- | -- | -- | -- | 1 |
| Perfume microcapsules (d₉₀ = 4 µm) | 0.2 | -- | -- | -- | -- |
| Perfume microcapsules | -- | 0.2 | -- | -- | -- |
| (d₉₀ = 15 µm) | | | | | |
| Perfume microcapsules (d₉₀ = 20 µm) | -- | -- | 0.2 | -- | 0.2 |
| Perfume microcapsules (d₉₀ = 25 µm) | -- | -- | -- | 0.2 | -- |
| Soil-Release-Polymer*** | 1.5 | 1.5 | 1.5 | 1.5 | 1.5 |
| Ethanol | 2 | 2 | 2 | 2 | 2 |
| Citric acid, Na salt | 2.8 | 2.8 | 2.8 | 2.8 | 2.8 |
| Silicone foam suppressant | 0,01 | 0,01 | 0,01 | 0,01 | 0,01 |
| Phosphonic acid, Na salt | 0.2 | 0.2 | 0.2 | 0.2 | 0.2 |
| Boric acid, Na salt | 1 | 1 | 1 | 1 | 1 |
| Fluorescent dye | 0.05 | 0.05 | 0.05 | 0.05 | 0.05 |
| Enzymes, dyes, preservatives | + | + | + | + | + |
| Water | To make 100 | | | | |

| | | | | | |
|---|---|---|---|---|---|
| * Structurant 1: Sovermol® 750 (purchased from Cognis) ** Structurant 2: Sovermol® 1102 (purchased from Cognis) *** Texcare® SRN 1706 (purchased from Clariant) | | | | | |

All liquid detergent compositions E1 to E5 gave rise to stable liquid detergent compositions in which the perfume microcapsules are evenly and stably suspended. The liquid detergent compositions E1 to E5 were even stable at 50 °C for at least 7 days.

All liquid detergent compositions E1 to E5 showed a good cleaning performance on textiles. Especially, no graying was observed. In addition, textiles treated with one of the liquid detergent compositions E1 to E5 exhibit an appealing and long-lasting smell.

## Claims

1. A structured liquid detergent composition comprising:
(a) surfactant at a level of from 5 up to 60 % by weight of said composition, said surfactant comprising nonionic surfactant, anionic surfactant, zwitterionic surfactant, cationic surfactant and mixtures thereof;
(b) a structurant at a level of from 0.1 to 5 % by weight of said composition, said structurant comprising a ring opening product of an epoxidized fatty ester with a dihydric or polyhydric alcohol;
(c) a plurality of encapsulated actives at a level of from 0.05 up to 1 % by weight of said composition, wherein the encapsulated actives have an d₉₀ value of below 50 µm.

2. The structured liquid detergent composition according to claim 1, wherein said epoxidized fatty ester is selected from the group consisting of epoxidized fatty acid esters, epoxidized fatty acid triglycerides and mixtures thereof.

3. The structured liquid detergent composition according to claim 2, wherein said epoxidized fatty acid triglyceride is selected from the group consisting of epoxidized beef tallow, epoxidized palm oil, epoxidized peanut oil, epoxidized rapeseed oil, epoxidized cottonseed oil, epoxidized soybean oil, epoxidized sunflower oil, epoxidized linseed oil and mixtures thereof.

4. The structured liquid detergent composition according to any of the claims 1 to 3, wherein said alcohol is selected from the group consisting of ethane-1,2-diol, propane-1,2-diol, propane-1,3-diol, butane-1,4-diol, hexane-1,6-diol, neopentyl glycol, trimethylol propane, glycerol, trimethylol ethane, pentaerythritol, sorbitol, alkyl glycols, oligomeric glycols, oligomeric glycerols and mixtures thereof.

5. The structured liquid detergent composition according to any of the claims 1 to 4., wherein said structurant is present at a level of from 0.15 up to 2 % by weight of said composition and, preferably, at a level of from 0.5 up to 1 % by weight of said composition.

6. The structured liquid detergent composition according to any of the claims 1 to 5, wherein said encapsulated actives have a d₉₀ value of below 40 µm, preferably a d₉₀ value of below 30 µm.

7. The structured liquid detergent composition according to any of the claims 1 to 6, wherein said encapsulated actives comprise friable microcapsules, moisture-activated microcapsules, heat- activated microcapsules, or combinations thereof.

8. The structured liquid detergent composition according to any of the claims 1 to 7, wherein said encapsulated actives comprise an active selected from the group consisting of perfumes, softening agents, anti-static agents, refreshing agents, anti-microbial agents, disinfecting agents, anti-wrinkle agents, malodor control agents, insect/pet repellents, skin/fabric conditioning agents, silicones, anti-microbials, brighteners, bleaches, antifoams, and combinations thereof.

9. The structured liquid detergent composition according to claim 8, wherein said encapsulated actives comprise a perfume as an active.

10. A method for manufacturing a structured liquid detergent composition comprising:
(a) surfactant at a level of from 5 up to 60 % by weight of said composition, said surfactant comprising nonionic surfactant, anionic surfactant, zwitterionic surfactant, cationic surfactant and mixtures thereof;
(b) a structurant at a level of from 0.1 to 5 % by weight of said composition, said structurant comprising a ring opening product of an epoxidized fatty ester with a dihydric or polyhydric alcohol;
(c) a plurality of encapsulated actives at a level of from 0.05 up to 1 % by weight of said composition,
wherein the structurant is added at a temperature above 50 °C.

## Patentansprüche

1. Strukturierte Flüssigwaschmittelzusammensetzung, umfassend:
(a) Tensid in einer Menge von 5 bis 60 Gew.-% der Zusammensetzung, wobei das Tensid nichtionisches Tensid, anionisches Tensid, zwitterionisches Tensid, kationisches Tensid und Mischungen daraus umfasst;
(b) ein Strukturant in einer Menge von 0,1 bis 5 Gew.-% der Zusammensetzung, wobei der Strukturant ein ringöffnendes Produkt eines epoxidierten Fettesters mit einem zweiwertigen oder mehrwertigen Alkohol umfasst;
(c) mehrere verkapselte Wirkstoffe in einer Menge von 0,05 bis 1 Gew.-% der Zusammensetzung, wobei die verkapselten Wirkstoffe einen D₉₀-Wert unter 50 µm aufweisen.

2. Strukturierte Flüssigwaschmittelzusammensetzung nach Anspruch 1, wobei der epoxidierte Fettester aus der Gruppe ausgewählt ist, die aus epoxidierten Fettsäureestern, epoxidierten Fettsäuretriglyceriden und Mischungen daraus besteht.

3. Strukturierte Flüssigwaschmittelzusammensetzung nach Anspruch 2, wobei das epoxidierte Fettsäuretriglycerid aus der Gruppe ausgewählt ist, die aus epoxidiertem Rindertalg, epoxidiertem Palmöl, epoxidiertem Erdnussöl, epoxidiertem Rapsöl, epoxidiertem Baumwollsamenöl, epoxidiertem Sojaöl, epoxidiertem Sonnenblumenöl, epoxidiertem Leinsamenöl und Mischungen daraus besteht.

4. Strukturierte Flüssigwaschmittelzusammensetzung nach einem der Ansprüche 1 bis 3, wobei der Alkohol aus der Gruppe ausgewählt ist, die aus Ethan-1,2-diol, Propan-1,2-diol, Propan-1,3-diol, Butan-1,4-diol, Hexan-1,6-diol, Neopentylglycol, Trimethylolpropan, Glycerin, Trimethylolethan, Pentaerythritol, Sorbitol, Alkylglycolen, oligomeren Glycolen, oligomeren Glycerinen und Mischungen daraus besteht.

5. Strukturierte Flüssigwaschmittelzusammensetzung nach einem der Ansprüche 1 bis 4, wobei der Strukturant in einer Menge von 0,15 bis 2 Gew.-% der Zusammensetzung und vorzugsweise in einer Menge von 0,5 bis 1 Gew.-% der Zusammensetzung vorliegt.

6. Strukturierte Flüssigwaschmittelzusammensetzung nach einem der Ansprüche 1 bis 5, wobei die verkapselten Wirkstoffe einen D₉₀-Wert von unter 40 µm, vorzugsweise einen D₉₀-Wert von unter 30 µm aufweisen.

7. Strukturierte Flüssigwaschmittelzusammensetzung nach einem der Ansprüche 1 bis 6, wobei die verkapselten Wirkstoffe brüchige Mikrokapseln, feuchtigkeitsaktivierte Mikrokapseln, wärmeaktivierte Mikrokapseln oder Kombinationen daraus umfassen.

8. Strukturierte Flüssigwaschmittelzusammensetzung nach einem der Ansprüche 1 bis 7, wobei die verkapselten Wirkstoffe einen Wirkstoff umfassen, der aus der Gruppe ausgewählt ist, die aus Duftstoffen, Weichmachern, Antistatika, Auffrischungsmitteln, antimikrobiellen Mitteln, Desinfektionsmitteln, Antifaltenmitteln, Schlechtgeruchkontrollmitteln, insekten-/haustierabweisenden Mitteln, Hautpflege-/Weichspülmitteln, Silikonen, antimikrobiellen Mitteln, Aufhellern, Bleichmitteln, Entschäumern und Kombinationen daraus besteht.

9. Strukturierte Flüssigwaschmittelzusammensetzung nach Anspruch 8, wobei die verkapselten Wirkstoffe einen Duftstoff als einen Wirkstoff umfassen.

10. Verfahren zum Herstellen einer strukturierten Flüssigwaschmittelzusammensetzung, umfassend:
(a) Tensid in einer Menge von 5 bis 60 Gew.-% der Zusammensetzung, wobei das Tensid nichtionisches Tensid, anionisches Tensid, zwitterionisches Tensid, kationisches Tensid und Mischungen daraus umfasst;
(b) ein Strukturant in einer Menge von 0,1 bis 5 Gew.-% der Zusammensetzung, wobei der Strukturant ein ringöffnendes Produkt eines epoxidierten Fettesters mit einem zweiwertigen oder mehrwertigen Alkohol umfasst;
(c) mehrere verkapselte Wirkstoffe in einer Menge von 0,05 bis 1 Gew.-% der Zusammensetzung,
wobei der Strukturant bei einer Temperatur über 50 °C hinzugefügt wird.

## Revendications

1. Composition de détergent liquide structurée comprenant :
(a) un tensioactif à une concentration de 5 jusqu'à 60 % en poids de ladite composition, ledit tensioactif comprenant un tensioactif non ionique, un tensioactif anionique, un tensioactif zwittérionique, un tensioactif cationique et leurs mélanges ;
(b) un structurant à une concentration de 0,1 à 5 % en poids de ladite composition, ledit structurant comprenant un produit à ouverture de cycle d'un ester gras époxydé avec un alcool dihydrique ou polyhydrique ;
(c) une pluralité d'agents actifs encapsulés à une concentration de 0,05 jusqu'à 1 % en poids de ladite composition, dans laquelle les agents actifs encapsulés ont une valeur D₉₀ en dessous de 50 µm.

2. Composition de détergent liquide structurée selon la revendication 1, dans laquelle ledit ester gras époxydé est sélectionné dans le groupe consistant en des esters d'acide gras époxydés, des triglycérides d'acide gras époxydés et leurs mélanges.

3. Composition de détergent liquide structurée selon la revendication 2, dans laquelle ledit triglycéride d'acide gras est sélectionné dans le groupe consistant en le suif de boeuf époxydé, l'huile de palme époxydée, l'huile d'arachide époxydée, l'huile de noix époxydée, l'huile de graine de coton époxydée, l'huile de soja époxydée, l'huile de tournesol époxydée, l'huile de graine de lin époxydée et leurs mélanges.

4. Composition de détergent liquide structurée selon l'une quelconque des revendications 1 à 3, dans laquelle ledit alcool est sélectionné dans le groupe consistant en l'éthane-1,2-diol, le propane-1,2-diol, le propane-1,3-diol, le butane-1,4-diol, l'hexane-1,6-diol, le néopentyl glycol, le triméthylol propane, le glycérol, le triméthylol éthane, le pentaérythritol, le sorbitol, les alkyl glycols, les glycols oligomériques, les glycérols oligomériques et leurs mélanges.

5. Composition de détergent liquide structurée selon l'une quelconque des revendications 1 à 4, dans laquelle ledit structurant est présent à une concentration de 0,15 jusqu'à 2 % en poids de ladite composition et, de préférence, à une concentration de 0,5 jusqu'à 1 % en poids de ladite composition.

6. Composition de détergent liquide structurée selon l'une quelconque des revendications 1 à 5, dans laquelle lesdits ingrédients actifs encapsulés ont une valeur d₉₀ en dessous de 40 pm, de préférence une valeur D₉₀ en dessous de 30 µm.

7. Composition de détergent liquide structurée selon l'une quelconque des revendications 1 à 6, dans laquelle lesdits ingrédients actifs encapsulés comprennent des microcapsules friables, des microcapsules activées par humidité, des microcapsules activées par chaleur, ou leurs combinaisons.

8. Composition de détergent liquide structurée selon l'une quelconque des revendications 1 à 7, dans laquelle lesdits ingrédients actifs encapsulés comprennent un ingrédient actif sélectionné dans le groupe consistant en les parfums, les agents adoucissants, les agents antistatiques, les agents rafraîchissants, les agents antimicrobiens, les agents désinfectants, les agents antirides, les agents désodorisants, les insectifuges/répulsifs pour animaux de compagnie, les revitalisants pour la peau/assouplissants pour tissu, les silicones, les antimicrobiens, les azureurs optiques, les agents de blanchiment, les antimousses, et leurs combinaisons.

9. Composition de détergent liquide structurée selon la revendication 8, dans laquelle lesdits ingrédients actifs encapsulés comprennent un parfum en tant qu'ingrédient actif.

10. Procédé de fabrication d'une composition de détergent liquide structurée comprenant :
(a) un tensioactif à une concentration de 5 jusqu'à 60 % en poids de ladite composition, ledit tensioactif comprenant un tensioactif non ionique, un tensioactif anionique, un tensioactif zwitterionique, un tensioactif cationique et leurs mélanges ;
(b) un structurant à une concentration de 0,1 à 5 % en poids de ladite composition, ledit structurant comprenant un produit à ouverture de cycle d'un ester gras époxydé avec un alcool dihydrique ou polyhydrique ;
(c) une pluralité d'agents actifs encapsulés à une concentration de 0,05 jusqu'à 1 % en poids de ladite composition,
dans lequel le structurant est ajouté à une température au-dessus de 50 °C.
